# EUROPEAN PATENT SPECIFICATION

(11) **EP 1 073 462 B1**
(45) Date of publication and mention of the grant of the patent: **22.11.2006**
(21) Application number: 99924871.9
(22) Date of filing: 27.04.1999
(51) Int. Cl.: A61K 39/29

(54) **Combined vaccine against Hepatitis A and Hepatitis B**
Kombinierte Impfstoffe gegen Hepatitis A und Hepatitis B
Vaccin combiné contre l'hépatite A et l'hépatite B

(30) Priority: 01.05.1998 GB 9809507
(43) Date of publication of application: 07.02.2001
(73) Proprietor: GlaxoSmithKline Biologicals S.A., 1330 Rixensart (BE)
(72) Inventor: ARTOIS, Claude, B-1330 Rixensart (BE); CLARK, Michael, J., B-1330 Rixensart (BE); THIRIART, Clothilde, B-1330 Rixensart (BE); THOELEN, Stefan, Gabriel, Jozef, B-1330 Rixensart (BE)
(74) Representative: Privett, Kathryn Louise
(86) International application number: PCT/EP1999/003024
(87) International publication number: WO 1999/056772

(56) References cited:
- WO-A-93/24148
- WO-A-96/26741
- F. AMBROSCH ET AL.: "CLINICAL AND IMMUNOLOGICAL INVESTIGATION OF A NEW COMBINED HEPATITIS A AND HEPATITIS B VACCINE." JOURNAL OF MEDICAL VIROLOGY, vol. 44, no. 4, December 1994 (1994-12), pages 452-456, XP000857078 NEW YORK, N.Y., US
- FREY S. ET AL.: "Interference of Antibody Production to Hepatitis B Surface Antigen in a Combination Hepatitis A/B Vaccine" THE JOURNAL OF INFECTIOUS DISEASES, vol. 180, - 1999 pages 2018-2022,

## Description

Vaccines for the prophylaxis of hepatitis A and hepatitis B infections are well known. For example the vaccine Engerix-B (Trade Mark) from SmithKline Beecham Biologicals is used to prevent Hepatitis B. This vaccine comprises hepatitis B surface antigen (specifically the 226 amino acid S- antigen described in Harford et al. in Postgraduate Medical Journal, 1987, 63 (Suppl. 2), 65-70) and is formulated using aluminium hydroxide as adjuvant. The vaccine Havrix (Trade Mark), also from SmithKline Beecham Biologicals can be used to prevent hepatitis A infections and is also formulated with aluminium hydroxide as adjuvant. This vaccine comprises an attenuated strain of the HM-175 Hepatitis A virus inactivated with formol (formaldehyde); see Andre et al [Prog Med. Virol. 1990, vol 37; p72-95]. The vaccine Twinrix (Trade Mark) which is a combination of the above hepatitis A and hepatitis B antigens may be used to protect against hepatitis A and hepatitis B simultaneously.

PCT application WO96/26741 (SmithKline Beecham) describes a vaccine formulation comprising a hepatitis B component, particularly hepatitis B surface antigen, in combination with aluminium phosphate and 3 de-O-acylated mono phosphoryl lipid A.

European patent 0 339 667 (Chemo Sero) describes the general concept of combining a hepatitis A antigen and a hepatitis B antigen to make a combination vaccine. In that specification it is stated that the adjuvant which is used is not critical: it must only be capable of enhancing the immune activity to a desired extent and not cause any side-effects. It is stated that aluminium gel may be used, in particular aluminium hydroxide gel and aluminium phosphate gel.

PCT application WO 93/24148 (SmithKline Beecham) describes the preparation of vaccines comprising hepatitis B surface antigen in which aluminium phosphate is used as adjuvant. Multivalent combination vaccines which may optionally contain a hepatitis A antigen, are described. The use of formol is not disclosed.

European Patent Number 0 633 784 (SmithKline Beecham) describes novel vaccine formulations comprising a hepatitis B component, particularly hepatitis B surface antigen, in combination with aluminium phosphate, and 3 de-O-acylated monophosphoryl lipid A.

Ambrosh F. et al (Journal of Medical Virology (1994)44:452-456) describe monovalent and combined vaccine formulations against hepatitis A and/or B comprising aluminium hydroxide, for immunization of young adults in a 3 dose regimen.

It has now been surprisingly found that vaccines comprising hepatitis B and/or hepatitis A antigens give exceptionally good results if the vaccine is formulated in a specific manner.

Using vaccine formulations according to the invention, it is possible to administer the vaccines in a 2 dose, rather than a 3 dose, regimen.

In a first aspect of the invention, there is provided the use of an aqueous vaccine composition comprising:
hepatitis B surface antigen;
an inactivated hepatitis A virus (HAV);
and formol,
in which there is 0.015-0.1 mg aluminium phosphate per µg hepatitis B surface antigen and 0.05 - 0.10 mg aluminium hydroxide per ml,
in the preparation of an immunoprotective hepatitis A plus B composition for administration to adolescents or children in a 2 dose schedule.

Preferably the aluminium phosphate is in the range 0.02 to 0.08mg per µg HBsAg.

The hepatitis A antigen is preferably the HM-175 strain used in the commercial product Havrix (SmithKline Beecham Biologicals).

The concentration of hepatitis A antigen in the vaccine formulation of the invention is preferably about 720-2880 EU units per ml. For the definition of EU units see Andre et al (1990) loc cit.

In the vaccine composition of the invention it is advantageous to add formol (formaldehyde) such that the formol concentration is 10-200 µg per ml.

Preferably the formol concentration is about 20-160 µg per ml.

Normally the hepatitis B antigen will be hepatitis B surface antigen (HBsAg). The preparation of Hepatitis B surface antigen (HBsAg) is well documented. See for example, Harford et al in Develop. Biol. Standard 54, page 125 (1983), Gregg et al in Biotechnology, 5, page 479 (1987), EP-A- 0 226 846, EP-A-0 299 108 and references therein.

As used herein the expression 'Hepatitis B surface antigen' or 'HBsAg' includes any HBsAg antigen or fragment thereof displaying the antigenicity of HBV surface antigen. It will be understood that in addition to the 226 amino acid sequence of the HBsAg S antigen (see Tiollais et al, Nature, 317, 489 (1985) and references therein) HBsAg as herein described may, if desired, contain all or part of a pre-S sequence as described in the above references and in EP-A-0 278 940. HBsAg as herein described can also refer to variants, for example the 'escape mutant' described in WO 91/14703. In a further aspect the HBsAg may comprise a protein described as SL* in European Patent Application Number 0 414 374, that is to say a protein, the amino acid sequence of which consists of parts of the amino acid sequence of the hepatitis B virus large (L) protein (ad or ay subtype), characterised in that the amino acid sequence of the protein consists of either:
(a) residues 12 - 52, followed by residues 133 - 145, followed by residues 175 - 400 of the said L protein; or
(b) residue 12, followed by residues 14 - 52, followed by residues 133 - 145, followed by residues 175 - 400 of the said L protein.

HBsAg may also refer to polypeptides described in EP 0 198 474 or EP 0 304 578.

Normally the HBsAg will be in particle form. It may comprise S protein alone or may be as composite particles, for example (L*,S) wherein L* is as defined above and S denotes the S-protein of hepatitis B surface antigen.

Preferably the HBsAg will be adsorbed on aluminium phosphate as described in WO93/24148.

Preferably the hepatitis B antigen is HBsAg S-antigen as used in the commercial product Engerix-B (SmithKline Beecham Biologicals).

The vaccine formulations of the present invention will contain an immunoprotective quantity of the antigens and may be prepared by conventional techniques. Vaccine preparation is generally described in New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S, Patent 4,372,945 and by Armor et al., U.S. Patent 4,474,757.

The vaccine compositions of the invention are preferably administered in a 0, 6 month schedule, that is to say a first dose at 0 months and a second dose at 6 months.

The following examples are for the purpose of further illustration

### EXAMPLES

### Example 1. Specific formulations

Specific formulations within the scope of the invention include the following:
a) A vaccine composition for administration to adolescents and/or children which comprises:
   20µg HBsAg
   720 EU HAV
   0.45mg Aluminium salt (0.4mg aluminium phosphate plus 0.05mg aluminium hydroxide)
   80µg formol
   in a 1 ml dose
b) A vaccine composition for administration to adolescents and/or children which comprises:
   20µg HBsAg
   720 EU HAV
   0.45mg Aluminium salt (0.4mg aluminium phosphate plus 0.05mg aluminium hydroxide)
   80µg formol
in a 0.5ml dose

### Example 2: Studies 'HAB 063, HAB071 and HAB 075'

### HAB 2 dose program in adolescents aged 11-18

### 2.1: Study HAB 063

Vaccine composition comprises:
720 HAV EU / 20µg HBs Ag
0.25µg Al Salt
40µg formol
In 0.5ml dose

Results are shown in Table 1.

**TABLE 1:**

| **HAB 063 study** | | | | | |
|---|---|---|---|---|---|
| **HAB 2-dose program in adolescents aged 11 -18years (0, 6month schedule)** | | | | | |
| **N=52** | | | | | |
| **Antibody** | | **PI (m1)** | **PI (m2)** | **PI (m6)** | **PII(m7)** |
| | Anti-HAV | | | | |
| S+ % | | 100 | 96.2 | 96.2 | 100 |
| GMT (EL. U/ml) | | 504 | 318 | 199 | 6874 |

| | Anti-HBs | | | | |
|---|---|---|---|---|---|
| S+% | | 51.9 | 75 | 94.2 | 98.1 |
| Seroprotection rate % | | 30.8 | 28.8 | 71.2 | 98.1 |
| GMT (mlU/ml) | | 11 | 7 | 19 | 4110 |

### 2.2: Study HAB 071

Two groups were used in this study.
Vaccine composition group 1:
720 HAV EU / 20µg HBs Ag
0.425mg Al Salt
40µg formol

In 0.5ml dose and at a schedule of 0, 6 months

Vaccine composition group 2 (Twinrix ^{™} Junior):
360 HAV EU /10 µg HBs Ag
0.225 mg Al Salt
40 µg formol

In 0.5ml dose and at a schedule of 0, 1, 6 months

**TABLE 2:**

| **HAB 071 study** | | | | | |
|---|---|---|---|---|---|
| **High Dose HAB (Group 1)** | | **PI (m1)** | **PI (M2)** | **PI (m6)** | **PI(m7)** |
| Schedule 0, 6 | | n=50 | n=48 | n=49 | n=49 |
| | Anti-HAV antibodies | | | | |
| S+% | | 100.0 | 100.0 | 100.0 | 100.0 |
| GMT (EL. U/ml) | | 641 | 347 | 161 | 8151 |

| | Anti-HBs antibodies | | | | |
|---|---|---|---|---|---|
| S+ % | | 74.0 | 70.8 | 85.7 | 100.0 |
| Seroprotoection rate % | | 40.0 | 27.1 | 57.1 | 100.0 |
| GMT (EL. U/ml) | | 12 | 9 | 17 | 4212 |
| | | | | | |

| **Twinrix** ^{**™**} **Junior (Group 2)** | | **PI (m1)** | **PI (M2)** | **PI (M2)** | **PI(m7)** |
|---|---|---|---|---|---|
| | Schedule 0, 1, 6 | n=49 | n=48 | n=48 | n=48 |
| | Anti-HAV antibodies | | | | |
| S+ % | | 95.9 | 100.0 | 97.9 | 100.0 |
| GMT (EL. U/ml) | | 336 | 793 | 233 | 6394 |

| | Anti-HBs antibodies | | | | |
|---|---|---|---|---|---|
| S+ % | | 61.2 | 97.9 | 100.0 | 100.0 |
| Seroprotoection rate % | | 36.7 | 83.3 | 97.9 | 100.0 |
| GMT (EL. U/ml) | | 12 | 36 | 202 | 6330 |

### 2.3 Study HAB 075

Two groups were used in this study.
Vaccine composition group 1 (Twinrix ^{™}):
720 HAV EU / 20µg HBs Ag
0.45mg Al Salt
80µg formol

In 1ml dose and at a schedule of 0, 6 months
N=67

Vaccine composition group 2:
1440 HAV EU / 40µg HBs Ag
0.85mg Al Salt
80µg formol

In 1 ml dose and at a schedule of 0, 6 months
N=55

**TABLE 3:**

| **HAB 075 study** | | | | | | |
|---|---|---|---|---|---|---|
| **Group** | | | **PI (m1)** | **PI (m2)** | **PI (m6)** | **PII(m7)** |
| **1** | | | | | | |
| | **Anti-HAV** | S+ % | 97.0 | 95.5 | 100.0 | 100.0 |
| | | GMT | 349 | 193 | 135 | 5646 |
| | **Anti-HBs** | S+% | 62.7 | 74.6 | 95.5 | 100.0 |
| | | SP rate % | 22.4 | 32.8 | 61.2 | 100.0 |
| | | GMT | 6 | 6 | 13 | 3046 |

| **2** | | | | | | |
|---|---|---|---|---|---|---|
| | **Anti-HAV** | S+% | 100.0 | 100.0 | 100.0 | 100.0 |
| | | GMT | 533 | 318 | 249 | 9565 |
| | **Anti-HBs** | S+ % | 67.3 | 85.5 | 94.5 | 100.0 |
| | | SP rate % | 32.7 | 41.8 | 72.7 | 100.0 |
| | | GMT | 9 | 9 | 26 | 3497 |

### Example 3: Study 'HAB 084'

### HAB 2 dose program in adolescents aged 12 - 15

Two groups were used in this study.
Vaccine composition group 1 (Twinrix^{™}):
720 HAV EU / 20µg HBs Ag
0.45mg Al Salt
80µg formol

In 1ml dose and at a schedule of 0, 6 months
Vaccine composition group 2 (Twinrix^{™} Junior):
360 HAV EU / 10µg HBs Ag
0.225mg Al Salt
40µg formol

In a 0.5ml dose and at a schedule of 0, 1, 6 months

**TABLE 4:**

| **HAB 084 Study** | | | | | | |
|---|---|---|---|---|---|---|
| Group | | | PI (m1) | PI (m2) | PI (m6) | PII (m7) |
| 1 | | | N=142 | N=142 | N=142 | N=142 |
| (0,6) | **Anti-HAV** | S+% | 99,3 | 100 | 100 | 100 |
| | | GMT | 348 | 244 | 178 | 5486 |
| | | | | | | |
| | **Anti-HBs** | S+% | 80,5 | 81 | 93 | 100 |
| | | SP rate % | 43 | 38 | 68 | 97,9 |
| | | GMT | 14 | 9 | 20 | 4948 |
| | | | | | | |

| | | | PI (m1) | PII (m2) | PII (m6) | PIII (m7) |
|---|---|---|---|---|---|---|
| 2 | | | N=148 | N=146 | N=147 | N=147 |
| (0, 1, 6) | **Anti-HAV** | S+% | 93.2 | 99.3 | 99.3 | 100 |
| | | GMT | 227 | 548 | 298 | 4174 |
| | | | | | | |
| | **Anti-HBs** | S+% | 58.1 | 95.9 | 99.3 | 100 |
| | | SP rate % | 29.1 | 85.6 | 98 | 100 |
| | | GMT | 9 | 42 | 305 | 5054 |
| | | | | | | |

### Example 4: Study 'HAB 076'

### HAB 2 dose program in children aged 1 -11 years

Vaccine composition comprises:
720 HAV EU / 20µg HBs Ag
0.45mg Al Salt
80µg formol

In 1ml dose at a schedule of 0, 1 month
Results are shown in Table 5.

**TABLE 5:**

| **HAB 076 Study** | | | | | |
|---|---|---|---|---|---|
| | | PI (m1) | PI (m2) | PI (m6) | PII (m7) |
| | | N=194 | N=201 | N=197 | N=199 |
| **Anti-HAV** | S+% | 99,5 | 98,5 | 98 | 100 |
| | GMT | 434 | 293 | 193 | 11543 |
| | | | | | |
| **Anti-HBs** | S+% | 72,7 | 89,1 | 93,9 | 100 |
| | SP rate % | 30,3 | 47,3 | 78,2 | 98,5 |
| | GMT | 8 | 11 | 34 | 8056 |
| | | | | | |

### SUMMARY

The clinical results shown in the above examples clearly indicate that a satisfactory immune response is obtained, both for hepatitis A and hepatitis B after the fill schedule, that is by Month 7, in children and adolescents.

## Claims

1. Use of an aqueous vaccine composition comprising :
hepatitis B surface antigen;
an inactivated hepatitis A virus (HAV);
and formol,
in which there is 0.015-0.1 mg aluminium phosphate per µg hepatitis B surface antigen and 0.05 - 0.10 mg aluminium hydroxide per ml,
for the manufacture of an immunoprotective hepatitis A plus B composition for administration to adolescents or children in a 2 dose schedule.

2. Use according to claim 1 in which the formal concentration is 10-200 µg per ml.

3. Use according to claim 2 in which the concentration of HAV is 720-2880 EU per ml.

4. Use according to claim 3 for administration to adolescents and/or children which comprises or consists of
20µg HBsAg
720 EU HAV
0.45 mg. Aluminium salt, consisting of 0.4 mg aluminium phosphate plus 0.05 mg aluminium hydroxide
80 µg formol
in a 1 ml dose

5. Use according to claim 3 for administration to adolescents and/or children which comprises or consists of:
20µg HBsAg
720 EU HAV
0.45 mg Aluminium salt, consisting of 0.4 mg aluminium phosphate plus 0.05 mg aluminium hydroxide
80 µg formol
in a 0.5 ml dose

6. Use according to any preceding claim wherein the hepatitis B surface antigen is the S-antigen.

7. Use according to any preceding claim in which the hepatitis A antigen is derived from the HM-175 strain.

8. Use according to any preceding claim in which the 2 doses are administered at month 0 and 6.

## Patentansprüche

1. Verwendung einer wässrigen Impfstoffzusammensetzung, die
Hepatitis B-Oberflächenantigen;
ein inaktiviertes Hepatitis A-Virus (HAV);
und Formol umfasst,
worin 0,015-0,1 mg Aluminiumphosphat
pro µg Hepatitis B-Oberflächenantigen und
0,05-0,10 mg Aluminiumhydroxid pro ml vorhanden sind,
zur Herstellung einer immunprotektiven Heptatitis A-plus B-Zusammensetzung zur Verabreichung an Jugendliche oder Kinder in einem 2-Dosis-Schema.

2. Verwendung gemäß Anspruch 1, worin die Formolkonzentration 10-200 µg pro ml beträgt.

3. Verwendung gemäß Anspruch 2, worin die Konzentration von HAV 720-880 EU pro ml beträgt.

4. Verwendung gemäß Anspruch 3 zur Verabreichung an Jugendliche und/oder Kinder, die Folgendes umfasst oder daraus besteht:
20 µg HBsAg,
720 EU HAV,
0,45 mg Aluminiumsalz, bestehend aus 0,4 mg Aluminiumphosphat plus 0,05 mg Aluminiumhydroxid,
80 µg Formol,
in einer 1 ml-Dosis.

5. Verwendung gemäß Anspruch 3 zur Verabreichung an Jugendliche und/oder Kinder, die Folgendes umfasst oder daraus besteht:
20 µg HBsAg,
720 EU HAV,
0,45 mg Aluminiumsalz, bestehend aus 0,4 mg Aluminiumphosphat plus 0,05 mg Aluminiumhydroxid,
80 µg Formol
in einer 0,5 ml-Dosis.

6. Verwendung gemäß jedem vorhergehenden Anspruch, worin das Hepatitis B-Oberflächenantigen das S-Antigen ist.

7. Verwendung gemäß jedem vorhergehenden Anspruch, worin das Hepatitis A-Antigen aus dem HM-175-Stamm abgeleitet ist.

8. Verwendung gemäß jedem vorhergehenden Anspruch, worin die 2 Dosen zum Monat 0 und 6 verabreicht werden.

## Revendications

1. Utilisation d'une composition aqueuse de vaccin comprenant :
un antigène HBs,
un virus inactivé de l'hépatite A (HAV),
et du formol,
dans laquelle il y a de 0,015 à 0,1 mg de phosphate d'aluminium par µg d'antigène HBs et de 0,05 à 0,10 mg d'hydroxyde d'aluminium par ml, pour la fabrication d'une composition d'immunoprotection contre l'hépatite A plus B pour une administration à des adolescents ou des enfants selon un planning de 2 doses.

2. Utilisation selon la revendication 1, dans laquelle la concentration de formol est de 10 à 200 µg par ml.

3. Utilisation selon la revendication 2, dans laquelle la concentration de virus inactivé de l'hépatite A (HAV) est de 720 à 2 880 UE par ml.

4. Utilisation selon la revendication 3, pour une administration à des adolescents et/ou des enfants qui comprend ou se compose de :
20 µg d'antigène HBs,
720 UE de virus inactivé de l'hépatite A (HAV),
0,45 mg de sel d'aluminium, consistant en 0,4 mg de phosphate d'aluminium plus 0,05 mg d'hydroxyde d'aluminium,
80 µg de formol,
dans une dose de 1 ml.

5. Utilisation selon la revendication 3, pour une administration à des adolescents et/ou des enfants qui comprend ou se compose de :
20 µg d'antigène HBs,
720 UE de virus inactivé de l'hépatite A (HAV),
0,45 mg de sel d'aluminium, consistant en 0,4 mg de phosphate d'aluminium plus 0,05 mg d' hydroxyde d'aluminium,
80 µg de formol,
dans une dose de 0,5 ml.

6. Utilisation selon l'une quelconque des revendications précédentes, dans laquelle l'antigène HBs est l'antigène S.

7. Utilisation selon l'une quelconque des revendications précédentes, dans laquelle l'antigène de l'hépatite A est dérivé à partir de la souche HM-175.

8. Utilisation selon l'une quelconque des revendications précédentes, dans laquelle les 2 doses sont administrées les mois 0 et 6.
